# EUROPEAN PATENT SPECIFICATION

(11) **EP 3 779 398 B1**
(45) Date of publication and mention of the grant of the patent: **04.09.2024**
(21) Application number: 19834971.4
(22) Date of filing: 02.07.2019
(51) Int. Cl.: G01N 1/22, G01N 1/00

(54) **DISSOLVED-GAS MEASUREMENT DEVICE**
VORRICHTUNG ZUR MESSUNG VON AUFGELÖSTEM GAS
DISPOSITIF DE MESURE DE GAZ DISSOUS

(30) Priority: 12.07.2018 JP 2018132058
(43) Date of publication of application: 17.02.2021
(73) Proprietor: Pureron Japan Co., Ltd., Iwaki-shi, Fukushima 970-1144 (JP)
(72) Inventor: NAKAJIMA, Hidetoshi, Iwaki-shi, Fukushima 970-1144 (JP); ABE, Yasuhiro, Iwaki-shi, Fukushima 973-8408 (JP); TAGUCHI, Koji, Iwaki-shi, Fukushima 973-8408 (JP)
(74) Representative: Schön, Christoph
(86) International application number: PCT/JP2019/026385
(87) International publication number: WO 2020/013041

(56) References cited:
- EP-A1- 3 045 900
- EP-A1- 3 312 584
- WO-A1-00/75634
- WO-A1-2008/067674
- JP-A- 2001 074 680
- JP-B1- 5 859 159
- US-A1- 2010 077 828

## Description

### Technical Field

The present invention relates to a dissolved-gas measurement device that measures a concentration of gas dissolved in aqueous solution of gas flowing through a pipe that a specific gas permeates.

### Background Art

There has been conventionally known a hydrogen-gas-in-oil detection device that causes hydrogen gas dissolved in insulating oil to permeate a permeable membrane and to transfer to a hydrogen gas chamber that is an enclosed space and detects an amount of the transferred hydrogen gas so as to measure a concentration of the hydrogen gas dissolved in the insulating oil (for example, see Patent Literature 1).

In Patent Literature 1, the device detects an amount of hydrogen gas that permeates the permeable membrane and transfers to the hydrogen gas chamber to measure a concentration of hydrogen gas dissolved in insulating oil by using a hydrogen gas detection sensor and discharges the hydrogen gas inside the hydrogen gas chamber from a ventilation hole by natural ventilation.
Patent Literature 2 discloses a continuous measurement method for hydrogen gas concentration and a hydrogen gas concentration measurement device, wherein a pipe is placed in such a manner as to come in contact with a hydrogen water, and from the hydrogen water, hydrogen gas is passed through the pipe and is introduced into a hollow part, a first gas is introduced into the hollow part by a gas supply pump, a second gas is extracted and exhausted from the hollow part by a gas exhaust pump, and hydrogen gas that has passed through the pipe and has been introduced into the hollow part from the hydrogen water, is circulated by a circulation pump through a gas supply conduit into the hollow part as to reach an equilibrium state between the concentration of hydrogen gas contained in the hydrogen water and the hydrogen gas concentration in the hollow part, and the hydrogen gas concentration in the hollow part is measured.
Patent Literature 3 discloses a method of sensing hydrogen gas in a liquid comprising: providing a measuring device comprising a hydrogen sensing chamber and a palladium hydrogen sensor mounted in contact with said chamber, positioning a hydrogen permeable membrane between the liquid and the hydrogen sensor and creating a headspace, selectively providing air to the sensor headspace, thereby bringing air into contact with said palladium sensor to refresh the palladium, after the palladium sensor is refreshed shutting off air flow to the sensor headspace, bringing liquid into said chamber, allowing hydrogen to pass through the membrane and reach equilibrium, and reading the hydrogen concentration. Patent Literature 4 discloses a housing including a gas separation membrane to a sensor unit capable of detecting the concentration of hydrogen gas such that liquid cannot permeate a closed space within the housing and only hydrogen gas dissolved in the liquid can permeate the closed space through the gas separation membrane, and detachably couples such a hydrogen sensor element to an opening of a container in which the liquid is held.

### Citation List

### Patent Literature

Patent Literature 1: Japanese Utility Model Application Laid-Open No. S57-164459
Patent Literature 2: EP 3 312 584 A1
Patent Literature 3: US 2010/077828 A1
Patent Literature 4: EP 3 045 900 A1

### Summary of Invention

### Technical Problem

It is considered that the device of Patent Literature 1 is used as a dissolved-gas measurement device that measures a concentration of a specific gas dissolved in aqueous solution of the gas flowing through a pipe that the specific gas permeates.

However, the device of Patent Literature 1 has a problem that, because the installation positions of the sensor and the ventilation hole are greatly different, a part of gas transferred to the enclosed space for storing gas that permeates the permeable membrane is not measured by the sensor and is discharged from the ventilation hole by natural ventilation, and the concentration of the specific gas dissolved in the aqueous solution of gas cannot be accurately measured.

In view of the problem of the conventional technology, the object of the present invention is to provide a dissolved-gas measurement device that can effectively measure the concentration of a specific gas dissolved in aqueous solution of the specific gas flowing through a pipe while continuing to discharge, by natural ventilation, gas in the enclosed space for storing the gas that permeates the pipe.

### Solution to Problem

A dissolved-gas measurement device according to the present invention as defined in claim 1, which measures a concentration of a specific gas dissolved in aqueous solution of the specific gas flowing through a pipe that the specific gas permeates, includes: a pipe holding part that fixes the dissolved-gas measurement device to the pipe; a gas storage part that is a space for storing gas that permeates the pipe; and a ventilation tube through which the gas stored in the gas storage part is discharged. The ventilation tube includes a gas sensor that measures the concentration of the specific gas flowing into the ventilation tube from the gas storage part, and a ventilation hole that discharges the gas to outside the ventilation tube. The gas sensor is arranged inside the ventilation tube to be located between the gas storage part and the ventilation hole.

The dissolved-gas measurement device of the present invention includes the pipe holding part that fixes the dissolved-gas measurement device to the pipe, the gas storage part that is a space for storing gas that permeates the pipe, and the ventilation tube through which the gas stored in the gas storage part is discharged.

The ventilation tube includes the gas sensor that measures the concentration of the specific gas flowing into the ventilation tube from the gas storage part and the ventilation hole that discharges gas outside the ventilation tube. The gas sensor is arranged inside the ventilation tube to be located between the gas storage part and the ventilation hole. As a result, because the specific gas that permeates the pipe and flows into the ventilation tube from the gas storage part is certainly measured by the gas sensor that measures the concentration of the specific gas when the specific gas is naturally discharged outside the ventilation tube, the concentration of gas dissolved in the aqueous solution of the gas flowing through the pipe can be effectively measured.

As described above, the present invention can provide the dissolved-gas measurement device that can effectively measure the concentration of gas dissolved in the aqueous solution of the gas flowing through the pipe while continuing to discharge, by natural ventilation, gas in the enclosed space for storing the gas that permeates the pipe.

In the dissolved-gas measurement device according to the present invention, the ventilation tube is arranged on a one-side end of the pipe holding part in a direction of the pipe.

In the dissolved-gas measurement device of the present invention, the ventilation tube is arranged on the one-side end of the pipe holding part in the direction of the pipe.

As a result, the dissolved-gas measurement device can be used to be fixed to the pipe so that the ventilation tube is located above the pipe when the specific gas is lighter than air, and the dissolved-gas measurement device can be used to be fixed to the pipe so that the ventilation tube is located below the pipe when the specific gas is heavier than air.

For that reason, because natural ventilation can be actively performed in view of the weight of the specific gas, the concentration of gas dissolved in the aqueous solution of the gas flowing through the pipe can be more accurately measured.

As described above, the present invention can provide the dissolved-gas measurement device that can more accurately measure the concentration of gas dissolved in the aqueous solution of the gas flowing through the pipe by actively performing natural ventilation of gas in the enclosed space for storing the gas that permeates the pipe.

### Brief Description of Drawings

FIG. 1 is a cross-sectional view illustrating a usage state of a dissolved-gas measurement device according to the present embodiment.
FIG. 2 is a cross-sectional view illustrating a usage state of the dissolved-gas measurement device according to the present embodiment.

### Description of Embodiments

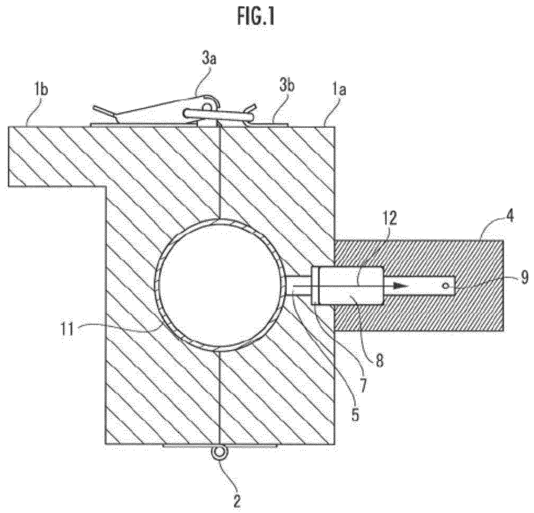

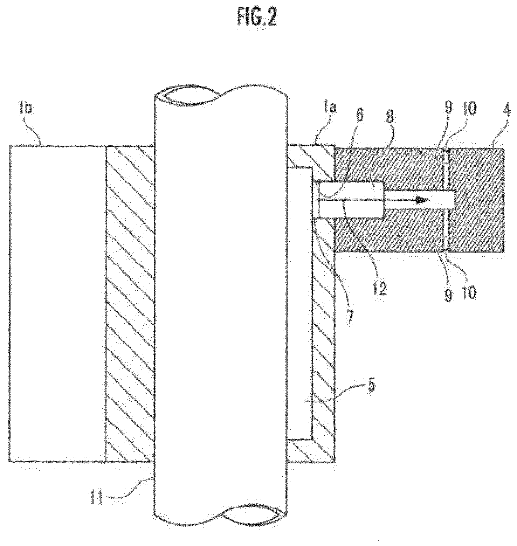

A configuration for realizing the present invention will be explained further in detail with reference to the accompanying FIGS. 1 and 2. Note that the same components are denoted by the same reference numerals and the description thereof may be omitted.

FIG. 1 is a planar cross-sectional view illustrating a usage state of a dissolved-gas measurement device according to the present embodiment. FIG. 2 is a front cross-sectional view illustrating the usage state of the dissolved-gas measurement device according to the present embodiment.

The dissolved-gas measurement device according to the present embodiment includes a pipe holding part 1, a hinge 2, a fixing part 3, and a ventilation tube 4.

The pipe holding part 1 is a component for fixing the dissolved-gas measurement device to a pipe 11, and is configured by a first pipe holding part 1a and a second pipe holding part 1b, for example. The first pipe holding part 1a includes a gas storage part 5 and an outlet 6.

The hinge 2 is fixed on one side of the first pipe holding part 1a and one side of the second pipe holding part 1b, and rotatably supports the first pipe holding part 1a and the second pipe holding part 1b.

The fixing part 3 is configured to be able to detachably fix the first pipe holding part 1a and the second pipe holding part 1b with the pipe 11 sandwiched therebetween, and is a catch clip configured by a locking part 3a and a locking receiving part 3b (part to be locked), for example. The fixing part 3 is fixed on other sides of the first pipe holding part 1a and the second pipe holding part 1b, which are sides opposite to the sides on which the hinge 2 is fixed. The fixing part 3 may include one component or may include two or more components.

The gas storage part 5 is an enclosed space for storing gas that permeates the pipe 11, and is open to the pipe 11 side. The edge of a portion of the gas storage part 5 that is open to the pipe 11 side is configured to have contact with the circumferential surface of the pipe 11 without any gap.

In an initial state where the dissolved-gas measurement device is fixed to the pipe 11, the gas storage part 5 is filled with gas that is substantially inert with respect to a specific gas whose concentration is to be measured.

In a state where the first pipe holding part 1a and the second pipe holding part 1b are rotated with the hinge 2 as a fulcrum to sandwich the pipe 11 such as a tube therebetween and are fixed by using the fixing part 3, a space surrounded by a part of the circumferential surface of the pipe 11 and the gas storage part 5 forms an enclosed space for storing gas that permeates the pipe 11.

The outlet 6 is a hole that discharges a mixed gas of the specific gas stored in the gas storage part 5 and the substantially inert gas with respect to the specific gas toward the ventilation tube 4. One side of the outlet is open on the surface of the gas storage part 5. The other side penetrates through the first pipe holding part 1a and is open on the opposite surface of the first pipe holding part 1a, and an O-ring 7 is fitted on its edge.

The ventilation tube 4 is arranged perpendicular to the direction of the pipe 11, for example, on the surface of the first pipe pressing portion 1a opposite to the side in contact with the pipe 11 so that the mixed gas flows into the ventilation tube 4 from the gas storage part 5. The ventilation tube 4 includes a gas sensor 8, a ventilation hole 9, and a ventilation filter 10.

The gas sensor 8 is a sensor configured to measure the concentration of the specific gas contained in the mixed gas. For example, the gas sensor has an annular structure and is arranged inside the ventilation tube 4 to be located between the gas storage part 5 and the ventilation hole 9. Moreover, the gas sensor 8 is fitted into a portion of the ventilation tube 4 that is open to the first pipe holding part 1a side, and the end of the gas sensor 8 on the first pipe holding part 1a side is arranged to have contact with the O-ring 7 without any gap.

The gas sensor 8 transmits a measurement result of the concentration of the specific gas contained in the mixed gas to a computer that collects and accumulates the data of the measurement result via a medium such as a cable not illustrated. Alternatively, the gas sensor 8 may transmit the measurement result of the concentration of the specific gas contained in the mixed gas to the computer through wireless communication.

The ventilation hole 9 is a hole that discharges, outside the ventilation tube 4 by natural ventilation, the mixed gas passing through the gas sensor 8 while moving in the direction indicated by the arrow 12 so as to be pushed out by the gas that permeates the pipe 11. For example, as illustrated in FIG. 2, the ventilation hole 9 penetrates through the ventilation tube 4 toward the upper and lower directions when fixing the dissolved-gas measurement device to the pipe 11.

Note that the penetration direction of the ventilation tube 4 is not limited to this. For example, the ventilation hole 9 may be configured to penetrate through the ventilation tube 4 toward the front or back direction when fixing the dissolved-gas measurement device to the pipe 11.

By naturally discharging the mixed gas from the gas storage part 5 in this way, the concentration of gas dissolved in the aqueous solution of the gas flowing through the pipe 11 and the concentration of the specific gas in the mixed gas in the gas storage part 5 are in equilibrium before long and the subsequent equilibrium state is maintained.

When the concentration of gas dissolved in the aqueous solution of the gas flowing through the pipe 11 and the concentration of the specific gas in the mixed gas in the gas storage part 5 reach equilibrium, the dissolved-gas measurement device measures the concentration of the specific gas contained in the mixed gas by using the gas sensor 8.

A time at which the concentration of gas dissolved in the aqueous solution of the gas flowing through the pipe 11 and the concentration of the specific gas in the mixed gas in the gas storage part 5 reaches equilibrium can be grasped by the measured concentration remaining constant for a predetermined time through the over-time measurements of the concentration of the specific gas in the mixed gas by the gas sensor 8, for example.

The ventilation filter 10 is a filter for preventing foreign matter from entering the ventilation tube 4 through the ventilation hole 9. The ventilation filter 10 prevents a failure of the gas sensor 8 due to the foreign matter.

The gas that permeates the pipe 11 is stored in the gas storage part 5, and passes through the outlet 6 and the gas sensor 8 while moving in the direction indicated by the arrow 12. The concentration of gas inside the pipe 11 can be measured without contamination of the aqueous solution of the gas flowing through the pipe 11 by measuring the concentration of the gas passing through the gas sensor 8 by the gas sensor 8. Then, after the concentration of this gas is measured by the gas sensor 8, this gas further moves in the ventilation tube 4 in the direction indicated by the arrow 12 and is discharged by natural ventilation outside the ventilation tube 4 through the ventilation hole 9.

According to the present invention including the configuration, the specific gas that permeates the pipe 11 and flows into the ventilation tube 4 from the gas storage part 5 is certainly measured by the gas sensor for measuring the concentration of the specific gas when being discharged by natural ventilation outside the ventilation tube 4, and thus the concentration of gas dissolved in the aqueous solution of the gas flowing through the pipe 11 can be effectively measured.

As described above, according to the present invention, it is possible to provide the dissolved-gas measurement device that can effectively measure the concentration of gas dissolved in the aqueous solution of the gas flowing through the pipe 11 while continuing to discharge, by natural ventilation, gas in the enclosed space for storing the gas that permeates the pipe 11.

Moreover, the ventilation tube 4 is arranged on a one-side end of the first pipe holding part 1a in the direction of the pipe 11 as illustrated in FIG. 2.

As a result, when the specific gas is lighter than air, the dissolved-gas measurement device can be used to be fixed to the pipe 11 so that the ventilation tube 4 is located above the pipe 11. When the specific gas is heavier than air, the dissolved-gas measurement device can be used to be fixed to the pipe 11 so that the ventilation tube 4 is located below the pipe 11.

For that reason, because natural ventilation can be actively performed in view of the weight of the specific gas, the concentration of gas dissolved in the aqueous solution of the gas flowing through the pipe 11 can be more accurately measured.

As described above, according to the present invention, it is possible to provide the dissolved-gas measurement device that can more accurately measure the concentration of gas dissolved in the aqueous solution of the gas flowing through the pipe 11 by actively performing natural ventilation of gas in the enclosed space for storing the gas that permeates the pipe 11.

For example, in the above embodiment, the pipe holding part 1 is configured by a pair of the pipe holding parts 1a and 1b that sandwich the pipe 11 therebetween and is configured to fix in a state where the pipe 11 is sandwiched by using the hinge 2 and the fixing part 3.

The pipe holding part 1 may be configured by one tubular component that is formed to wrap around the outer circumference of the pipe 11. In this case, the dissolved-gas measurement device does not include the hinge 2 and the fixing part 3.

### Description of Reference Numerals

1a ... first pipe holding part,
1b ... second pipe holding part,
2 ... hinge,
3 ... fixing part,
4 ... ventilation tube,
5 ... gas storage part,
6 ... outlet,
7 ... O-ring,
8 ... gas sensor,
9 ... ventilation hole,
10 ... ventilation filter,
11 ... pipe

## Claims

1. A dissolved-gas measurement device that measures a concentration of a specific gas dissolved in aqueous solution of the specific gas flowing through a pipe (11) that the specific gas permeates, the dissolved-gas measurement device comprising:
a pipe holding part comprising first and second pipe holding parts (1a, 1b) that fix the dissolved-gas measurement device to the pipe (11);
a gas storage part (5) that is adapted to store gas that permeates the pipe (11); and
a ventilation tube (4) through which the gas stored in the gas storage part (5) is discharged,
the ventilation tube (4) including a gas sensor (8) that measures the concentration of the specific gas flowing into the ventilation tube (4) from the gas storage part (5), and a ventilation hole (9) that discharges the gas to outside the ventilation tube (4), **characterized in that**
the ventilation tube (4) is arranged on one-side end of the pipe holding parts (1a, 1b) in a direction of the pipe and perpendicular to the direction of the pipe (11) on a surface of the first pipe pressing portion (1a) opposite to a side in contact with the pipe (11) so that mixed gas flows into the ventilation tube (4) from the gas storage part (5), said ventilation tube (4) being configured to be located above the pipe (11) when the specific gas is lighter than air and configured to be located below the pipe (11) when the specific gas is heavier than air, wherein
the gas sensor (8) is arranged inside the ventilation tube to be located between the gas storage part (5) and the ventilation hole (9).

## Patentansprüche

1. Messvorrichtung für gelöste Gase, die die Konzentration eines speziellen Gases misst, das in einer wässrigen Lösung des speziellen Gases gelöst ist, die durch ein Rohr (11) fließt, durch das das spezielle Gas permeiert, wobei die Messvorrichtung für gelöste Gase Folgendes umfasst:
ein Rohrhalteteil, das ein erstes und ein zweites Rohrhalteteil (1a, 1b) umfasst, die die Messvorrichtung für gelöste Gase an dem Rohr (11) befestigen,
ein Gasspeicherteil (5), das dazu geeignet ist, Gas zu speichern, das durch das Rohr (11) permeiert,
ein Entlüftungsrohr (4), durch das das in dem Gasspeicherteil (5) gespeicherte Gas abgelassen wird,
wobei das Entlüftungsrohr (4) einen Gassensor (8), der die Konzentration des speziellen Gases, das aus dem Gasspeicherteil (5) in das Entlüftungsrohr (4) strömt, misst, und ein Entlüftungsloch (9), das das Gas nach außen aus dem Entlüftungsrohr (4) ableitet, umfasst,
**dadurch gekennzeichnet, dass**
das Entlüftungsrohr (4) an einem seitlichen Ende der Rohrhalteteile (1a, 1b) in einer Richtung des Rohrs und senkrecht zur Richtung des Rohrs (11) auf einer Oberfläche des ersten Rohrpressabschnitts (1a) gegenüberliegend zu einer Seite, die sich in Kontakt mit dem Rohr (11) befindet, angeordnet ist, so dass gemischtes Gas von dem Gasspeicherteil (5) in das Entlüftungsrohr (4) strömt, wobei das Entlüftungsrohr (4) so konfiguriert ist, dass es oberhalb des Rohrs (11) angeordnet ist, wenn das spezielle Gas leichter als Luft ist, und so konfiguriert ist, dass es unterhalb des Rohrs (11) angeordnet ist, wenn das spezielle Gas schwerer als Luft ist, wobei
der Gassensor (8) im Inneren des Entlüftungsrohrs so angeordnet ist, dass er sich zwischen dem Gasspeicherteil (5) und dem Entlüftungsloch (9) befindet.

## Revendications

1. Dispositif de mesure de gaz dissous qui mesure une concentration d'un gaz spécifique dissous dans une solution aqueuse du gaz spécifique s'écoulant à travers un tube (11) que le gaz spécifique pénètre, le dispositif de mesure de gaz spécifique comprenant :
un élément de retenue du tube comprenant des première et seconde pièces de retenue du tube (1a, 1b) qui fixent le dispositif de mesure de gaz dissous au tube (11) ;
un élément de stockage de gaz (5) qui est apte à stocker du gaz qui pénètre le tube (11) ; et
un tube de ventilation (4) à travers lequel le gaz stocké dans l'élément de stockage de gaz (5) est déchargé,
le tube de ventilation (4) incluant un capteur de gaz (8) qui mesure la concentration du gaz spécifique s'écoulant dans le tube de ventilation (4) depuis l'élément de stockage de gaz (5), et un trou de ventilation (9) qui décharge le gaz vers l'extérieur du tube de ventilation (4), **caractérisé en ce que**
le tube de ventilation (4) est disposé sur une extrémité unilatérale des pièces de retenue du tube (1a, 1b) dans un sens du tube et perpendiculairement au sens du tube (11) sur une surface de la première pièce de compression du tube (1a) opposée à une face en contact avec le tube (11), de sorte que du gaz mélangé s'écoule dans le tube de ventilation (4) depuis l'élément de stockage de gaz (5), ledit tube de ventilation (4) étant configuré pour être situé au-dessus du tube (11) lorsque le gaz spécifique est plus léger que l'air et configuré pour être situé en dessous du tube (11) lorsque le gaz spécifique est plus lourd que l'air,
le capteur de gaz (8) étant disposé à l'intérieur du tube de ventilation afin d'être situé entre l'élément de stockage de gaz (5) et le trou de ventilation (9).
